Europäisches Patentamt

**European Patent Office**

**Office européen des brevets**

(19)

(11) Publication number: **0 081 823**
A1

(12)

# EUROPEAN PATENT APPLICATION

(21) Application number: **82111449.3**

(22) Date of filing: **10.12.82**

(51) Int. Cl.³: **A 61 K 31/485,** A 61 K 31/62, A 61 K 45/06

(30) Priority: **10.12.81 US 329550**

(43) Date of publication of application: **22.06.83** Bulletin 83/25

(84) Designated Contracting States: **BE CH DE FR GB IT LI NL SE**

(71) Applicant: **NELSON RESEARCH & DEVELOPMENT COMPANY, 19732 MacArthur Boulevard, Irvine, CA 92715 (US)**

(72) Inventor: **Nelson, Eric L., 12122 Sky Lane, Santa Ana California 92705 (US)**

(74) Representative: **Kraus, Walter, Dr. et al, Patentanwälte Dres. Kraus & Weisert Irmgardstrasse 15, D-8000 München 71 (DE)**

(54) Composition for reducing menstrual pain.

(57) A composition for treating dysmenorrhea in a human female having dysmenorrhea comprising an effective, menstrual pain reducing amount of dextromethorphan, preferably as the hydrobromide.

## COMPOSITION FOR REDUCING MENSTRUAL PAIN

### BACKGROUND OF THE INVENTION

#### 1. Field of the Invention

The invention relates to a composition for treatment of dysmenorrhea. More particularly the invention relates to a composition for temporarily reducing pain and discomfort in human females associated with menstruation.

#### 2. Background of the Prior Art

Dysmenorrhea is pain at the time of menstruation for which no organic cause can be found. It may occur shortly after menarche (primary dysmenorrhea) or later in life (secondary dysmenorrhea).

Dysmenorrhea is characterized by lower abdominal cramping accompanied by headache, backache, nausea and sometimes vomiting. The etiology of primary dysmenorrhea is unknown, but it is generally associated with hormonal imbalance and release of prostaglandins though it also can be aggravated by emotional factors.

Typical treatment for dysmenorrhea is symptomatic relief of pain and cramping with combinations of analgesics (including prostaglandin synthetase inhibitors such as aspirin, indomethacin and ibuprofen), diuretics, antihistamines and antispasmodics.

### SUMMARY OF THE INVENTION

Dextromethorphan is an old compound used heretofore as an antitussive. It is marketed in a wide variety of "over-the-counter" (OTC) and prescription (Rx) products for relief of cough, typically as the hydrobromide. It is described in the art consistently as having no significant analgesic activity.

Notwithstanding the long established belief that dextromethorphan has no significant analgesic activity, it has now been discovered that dextromethorphan is useful in the treatment of dysmenorrhea in the temporary reduction of menstrual pain and discomfort.

More particularly, the invention relates to a method of temporarily reducing pain and discomfort associated with dysmenorrhea comprising administering to a human female having dysmenorrhea an effective, menstrual pain reducing amount of dextromethorphan or a pharmaceutically active salt thereof. The preferred active compound is dextromethorphan hydrobromide.

The invention further relates to pharmaceutical compositions comprising an effective, menstrual pain reducing amount of dextromethorphan and preferably dextromethorphan hydrobromide in combination with one or more of the following:

1) a conventional diuretic such as, for example, Pamabrom (2-amino-2-methyl-1-propanol 8-bromo-Theophyllinate); or

2) a conventional antihistaminic, such as, for example Pyrilamine maleate; or

3) a conventional antispasmodic, such as, for example, cinnamedrine hydrochloride; or

4) a conventional analgesic, such as, for example, aspirin or acetaminophen, indomethacin, ibuprofen or naproxen.

## DETAILED DESCRIPTION OF THE INVENTION

Dextromethorphan (d-3-methoxy-N-methylmorphinan) is the d-isomer of the codeine analog of levorphanol; however, unlike the l-isomer, it has consistently been reported by the prior art as having no significant analgesic properties. The compound is well known in the art as a cough suppressant (antitussive) and is commercially available; e.g., U.S. Patent No. 2,676,177 and Häfliger et al, Helv. Chim. Acta 39, 2053 (1956). The hydrobromide salt of dextromethorphan is widely commercially used as an "over-the-counter" (OTC) orally administered antitussive. It is also used as an antitussive in combination with antihistamines in prescription (Rx) products for cold remedies.

Dextromethorphan may be used in the present invention in daily dosage amounts between about 5 mg and 500 mg and preferably between about 10 mg and about 200 mg depending on the age and weight of the human female to be treated and the severity of the menstrual pain to be treated. A typical daily dosage amount varies between about 10 mg and about 200 mg and preferably between about 20 mg and 100 mg. For example, a typical dosage amount of dextromethorphan hydrobromide effective in temporarily reducing menstrual pain in an adult human female would be about 10 mg to about 50 mg administered in equal doses 1 to 4 times per day.

Antihistamines which may be used in combination with dextromethorphan in the present invention are conventional antihistamines which are well known and widely used in combination with conventional analgesics, such as, for example, aspirin and acetaminophen in the treatment of menstrual pain. Typical antihistamines include, for example, Pyrilamine maleate, methapyrilene fumarate and phenindamine hydrogen tartrate. Antihistamines may be used in the present invention in combination with dextromethorphan in dosage

- 4 -                                    0081823

amounts conventionally used for treatment of menstrual pain and discomfort. For example, pyrilamine maleate is typically used in amounts between about 25 and about 100 mg per daily dosage.

Diuretics which may be used in combination with dextromethorphan in the present invention are conventional diuretics which are widely known and widely used in combinations with conventional analgesics in the treatment of menstrual pain. Typical OTC diuretics include Pamabrom caffeine and ammonium chloride; prescription diuretics include hydrochlorothiazide. Diuretics may be used in the present invention in combination with dextromethorphan in dosage amounts conventionally used for treatment of menstrual pain and discomfort. For example, Pamabrom is typically used in amounts between about 25 mg and about 200 mg per daily dosage.

Antispasmodics, such as, cinnamedrine hydrochloride may also be used in the treatment of menstrual pain and discomfort in conventional dosage amounts. For example, cinnamedrine hydrochloride is typically used in amounts between about 15 mg and about 120 mg per daily dosage.

For therapeutic use, dextromethorphan will normally be administered as a pharmaceutical composition in the basic form or in the form of an addition salt with a pharmaceutically acceptable acid and in association with a pharmaceutical carrier therefor. Such addition salts include those with hydrochloric, hydrobromic, hydriodic, sulphuric and maleic acids and preferably hydrobromic.

Other pharmacologically active compounds may, in certain cases, be included in the composition. Advantageously, the composition will be made up in a dosage unit form appropriate to the desired mode of administration, for example, as a tablet, capsule, sustained release type, oral suspension, or in a suitable formulation for conventional or sustained release topical administration.

The pharmaceutical compositions may be in a form suitable for oral use, for example, as tablets, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide a pharmaceutically elegant and palatable preparation. Tablets contain the active ingredient in admixture with non-toxic pharmaceutically acceptable excipients which are suitable for manufacture of tablets. These excipients may be, inert diluents, for example calcium carbonate, sodium carbonate, lactose, calcium phosphate or sodium phosphate; granulating and disintegrating agents, for example, maize starch, or alginic acid; binding agents for example, starch, gelatine or acacia, and lubricating agents, for example, magnesium stearate or stearic acid. The tablets may be uncoated or they may be coated by known techniques to to delay disintegration and absorption in the gastrointestinal tract and thereby provide a sustained action over a longer period.

Formulations for oral use may also be presented as hard gelatine capsules wherein the active ingredient is mixed with an inert solid diluent, for example, calcium carbonate, calcium phosphate or kaolin, or as soft gelatine capsules wherein the active ingredient is mixed with an oil medium, for example, arachis oil, liquid paraffin or olive oil.

Aqueous suspensions contain the active ingredients in admixture with excipients suitable for the manufacture of aqueous suspensions. Such excipients are suspending agents, for example, sodium carboxymethyl cellulose, methylcellulose, hydroxypropylmethylcellulose, sodium alginate, polyvinylpyrrolidone, gum tragacanth and gum acacia; dispersing or wetting agents may be a naturally occurring phosphatide, for example, lecithin, or condensation products of an alkylene oxide with fatty acids, for example, polyoxyethylene stearate, or condensation products of ethylene oxide with long chain aliphatic alcohols, for example heptadecaethyleneoxycetanol, or condensation products of ethylene oxide with partial esters derived from fatty acids and a hexitol, for example, polyoxyethylene sorbitol monooleate, or condensation product of ethylene oxide with partial esters derived from fatty acids and hexitol anhydrides, for example, polyoxyethylene sorbitan monooleate. The said aqueous suspensions may also contain one or more preservatives, for example ethyl- or n-propyl- p-hydroxy benzoate, one or more coloring agents, one or more flavoring agents and one or more sweetening agents, such as sucrose, saccharin, or sodium or calcium cyclamate.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present. Syrups and elixirs may be formulated with sweetening agents, for example glycerol, sorbitol or sucrose. Such formulations may also contain a demulcent, a preservative and flavoring and coloring agents.

The pharmaceutical compositions may be tableted or otherwise formulated so that for every 100 parts by weight of the composition there are present between 5 and 95 parts by weight of the active ingredient and preferably between 25 and 85 parts by weight of the active ingredient. The dosage unit form will generally contain between about 10 mg and about 500 mg of the active ingredients of the formula stated above.

From the foregoing formulation discussion, it is apparent that the composition of this invention is preferably administered topically or orally.

The scientific basis of the descovery set forth herein is not fully understood; however, it is believed that dextromethorphan is able to exert its pain reducing activity by acting upon specific pain mediating receptors in the body associated with endogenous peptides recently discovered to be involved in the mediation of pain, which peptides are known as enkephalins.

The following examples are shown for the purpose of illustration only and should not be deemed as limiting the scope of the invention.

EXAMPLE I

Female patient, aged 22 with a history of severe menstrual cramps, that did not respond to aspirin alone was given capsules containing 20 mg each of dextromethorphan hydrobromide and advised to take one capsule at the first sign of cramps or low back pain associated with her next menstrual period. This dose was to be repeated every 3-4 hours. No other medication was to be taken.

The patient reported that her subsequent menstrual period was initiated with severe cramps and low back pain. After enduring the pain for approximately 30 minutes and noting that the severity of pain was increasing the patient swallowed a 20 mg capsule of dextromethorphan hydrobromide with water. Within 15 minutes the pain began to subside and by 30 minutes was lessened considerably.

Three hours after the initiation of pain the severity increased and the patient took a second 20 mg capsule. Once again the pain was reduced to a bearable level but did not disappear entirely.

Symptoms of severe pain continued to reoccur every 3-4 hours for the next 36 hours and each time the pain was substantially reduced by a 20 mg capsule of dextromethorphan hydrobromide.

EXAMPLE II

At her next menstrual period the patient described in Example I was instructed to take one 20 mg capsule of dextromethorphan hydrobromide in combination with two 250 mg tablets of aspirin. This combination was directed at relieving both prostaglandin and non-prostaglandin induced pain.

At the first onset of pain the patient took a 20 mg oral capsule containing dextromethorphan hydrobromide and the two 250 mg aspirin tablets. The patient reported that the pain was completely relieved within 30 minutes and did not reappear for four hours.

The regimen was repeated at 4 hour intervals for 18 hours and the patient reported that pain did not re-occur until the following day. At that time the dosage was repeated twice over 8 hours and no further pain was reported.

EXAMPLE III

A second patient aged 27 with a history of menstrual cramps, nausea and associated skin disorders (acne), was instructed to take two tablets containing 10 mg each of dextromethorphan hydrobromide at the first sign of pain associated with her menstrual period.

The patient reported that pain was relieved within 15 minutes and did not become severe again for three hours. The regimen was repeated every 3-4 hours and pain was maintained at modest levels.

EXAMPLE IV

At her next menstrual period the patient described in Example III was given medication containing 10 mg dextromethorphan hydrobromide and 250 mg aspirin. She was instructed to take the medication at the beginning of her menstrual period, to continue to take the same dosage amount every four hours until bedtime, and to repeat the regimen the next day if pain continued.

The patient reported that pain was relieved initially within 30 minutes and did not reoccur until the following day at which time she repeated the treatment regimen every four hours until bedtime and no further pain was reported.

CLAIMS

1. A pharmaceutical composition comprising an effective, menstrual pain reducing amount of dextromethorphan or a pharmaceutically acceptable salt thereof and an effective amount of a diuretic.

2. The composition of Claim 1 wherein the diuretic is selected from the group consisting of Pamabrom and caffeine.

3. The composition of Claim 1 additionally containing an effective amount of an antihistamine.

4. The composition of Claim 3 wherein the antihistamine is selected from the group consisting of Pyrilamine maleate, methapyrilene fumarate and phenindamine hydrogen tartrate.

5. A pharmaceutical composition comprising an effective, menstrual pain reducing amount of dextromethorphan or a pharmaceutically acceptable salt thereof and an effective amount of an antispasmodic.

6. The composition of Claim 5 wherein the antispasmodic is cinnamedrine hydrochloride.

7. A pharmaceutical composition comprising an effective, menstrual pain reducing amount of dextromethorphan or a pharmaceutically acceptable salt thereof and an effective amount of an analgesic.

8. The composition of Claim 7 wherein the analgesic is selected from the group consisting of aspirin, acetaminophen, indomethacin, ibuprofen and naproxen.

9. A pharmaceutical composition comprising an effective, menstrual pain reducing amount of dextromethorphan hydrobromide, an effective amount of Pamabrom and an effective amount of Pyrilamine maleate.

0081823

10. A pharmaceutical composition comprising an effective, menstrual pain reducing amount of dextromethorphan hydrobromide, an effective amount of caffeine and an effective amount of cinnamedrine hydrochloride.

11. A pharmaceutical composition comprising an effective, menstrual pain reducing amount of dextromethorphan hydrobromide and an effective amount of aspirin.

12. A pharmaceutical composition comprising an effective, menstrual pain reducing amount of dextromethorphan or a pharmaceutically acceptable salt thereof for use in temporarily reducing pain and discomfort associated with dysmenorrhea by administering it to a human female having dysmenorrhea.

13. The composition of Claim 12 wherein the dextromethorphan is administered in a daily dosage regimen of about 5 mg to about 500 mg.

14. The composition of Claim 12 wherein the dextromethorphan is administered in a daily dosage regimen of about 10 mg to about 200 mg.

15. The composition of Claim 12 wherein the dextromethorphan is administered in the form of its hydrobromide salt.

16. The composition of Claim 15 wherein the dextromethorphan hydrobromide is administered orally.

17. The composition of Claim 12 wherein the dextromethorphan is administered in an oral dosage form selected from the group consisting of a tablet, capsule, lozenge, syrup, suspension and elixir.

**0081823**

Application number

# EUROPEAN SEARCH REPORT

**European Patent Office**

EP 82 11 1449

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | UNLISTED DRUGS, vol. 24, no. 3, March 1972, page 44f, Chatham, New Jersey (USA); <br> *Page 44f: "TONECOL"* | 1-4,7 | A 61 K 31/485 <br> A 61 K 31/62 <br> A 61 K 45/06 |
| | --- | | |
| X | UNLISTED DRUGS, vol. 22, no. 10, October 1970, page 154g, Chatham, New Jersey (USA); <br> *Page 154g: "S-FC"* | 1-4,7 | |
| | --- | | |
| X | UNLISTED DRUGS, vol. 32, no. 7, July 1980, page 105n, Chatham, New Jersey (USA); <br> *Page 105n: "CODEC"* | 1-4,7-8 | |
| | --- | | |
| X | H.D.FEIN: "Modern Drug Encylopedia and Therapeutic Index", 8th Edition, 1961, The Reuben H. Donnelley Corporation, New York (USA); <br> *Page 1096: "ROMILAR CF"* | 1-4,7-8 | TECHNICAL FIELDS SEARCHED (Int. Cl. ³) |
| | --- | | A 61 K <br> C 07 D |
| Y | M.NEGWER: "Organische-chemische Arzneimittel und ihre Synonyma", 5th Edition, vol. II, 1978, Akademie-Verlag, Berlin (DE); <br> *Page 677, nr. 3724 "Dextromethorphan"* | 12-17 | |
| | ---     -/- | | |

The present search report has been drawn up for all claims

| Place of search THE HAGUE | Date of completion of the search 09-03-1983 | Examiner BRINKMANN C. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

**EUROPEAN SEARCH REPORT**

**0081823**
Application number

EP 82 11 1449

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. 3) |
|---|---|---|---|
| X | GB-A-2 074 026 (NELSON RESEARCH & DEVELOPMENT COMPANY) *Page 3, lines 70-120; claims 1-13* | 12-17 | |
| X | FR-M- 7 195 (F.HOFFMANN-LA ROCHE & CIE.) *Page 5, left-hand column, abstract* | 7-8 | |
| X | FR-M- 2 320 (F.HOFFMANN-LA ROCHE & CIE.) *Page 2, right-hand column, abstract* | 7 | |
| X | ROTE LISTE, 1977/78, Editor Cantor, Aulendorf/Württ (DE); *Nr. 44 058B: "PINIGRIPPIN"* | 1-4,9 | TECHNICAL FIELDS SEARCHED (Int. Cl. 3) |
| A | UNLISTED DRUGS, vol. 25, no. 11, November 1973, Chatham, New Jersey (USA); *Page 179i: "SUNRIL"* | 1-4,9 | |
| A | H.D.FEIN: "Modern Drug Encyclopedia and Therapeutic Index", 8th Edition, 1961, The Reuben H. Donnelley Corporation, New York (USA); *Page 1113: "SANBROM"* | 1-4,9 | |
| D,A | US-A-2 676 177 (OTTO SCHNIDER) | 1-17 | |

The present search report has been drawn up for all claims

| Place of search THE HAGUE | Date of completion of the search 09-03-1983 | Examiner BRINKMANN C. |
|---|---|---|